# EUROPEAN PATENT SPECIFICATION

(11) **EP 3 043 746 B1**
(45) Date of publication and mention of the grant of the patent: **08.11.2017**
(21) Application number: 14766793.5
(22) Date of filing: 12.09.2014
(51) Int. Cl.: A61F 2/24

(54) **VASCULAR IMPLANT**
GEFÄSSIMPLANTAT
IMPLANT VASCULAIRE

(30) Priority: 13.09.2013 GB 201316349
(43) Date of publication of application: 20.07.2016
(73) Proprietor: UCL BUSINESS PLC, London W1T 4TP (GB)
(72) Inventor: BURRIESCI, Gaetano, London Greater London EC1V 8EL (GB); TZAMTZIS, Spyridon, London Greater London EC1V 8EL (GB); MULLEN, Michael John, London Greater London W1G 8PH (GB); SEIFALIAN, Alexander, London Greater London NW3 2QG (GB); YAP, John, London Greater London W1G 8PH (GB)
(74) Representative: J A Kemp
(86) International application number: PCT/GB2014/052785
(87) International publication number: WO 2015/036790

(56) References cited:
- WO-A1-2006/009690
- WO-A1-2010/037141
- WO-A2-2006/005015
- WO-A2-2010/121076
- US-A1- 2005 137 682
- US-A1- 2013 144 378

## Description

The present invention relates to a vascular implant, such as, but not limited to, a prosthetic heart valve.

Prosthetic heart valves have been an area of considerable research in recent years. Typically, the prosthetic valve comprises two elements: a support structure comprising a generally tubular framework surrounding a flow passage; and a flow control structure provided in the lumen of the support structure and providing the one-way valve action to permit blood flow in one direction through the valve, but preventing blood flow in the reverse direction.

However, a major problem with such implants has been the occurrence of mild to moderate paravalvular leakage. This can be particularly severe in the case of sutureless, percutaneous and transcatheter valves, that are not typically sutured to the host tissues, for which leakage has been shown to drastically reduce the hemodynamic efficiency of the valve. This can result in a significant increase in the load on the heart because any blood that leaks in the reverse flow direction, around the closed valve, does not contribute to the useful circulatory output of the heart.

WO 2006/009690 A1 discloses an everting heart valve.

Various proposals have been put forward to attempt to pack material to seal between the outside of the valve and the surrounding anatomy, but have not been entirely satisfactory, which is a problem.

A further problem is that it is undesirable for the implant to apply a high radial force to the surrounding anatomy to attempt to form a seal to avoid paravalvular leakage.

Another problem is that it is desirable for implants to be retrievable, so any sealing means that permanently hooks into the surrounding anatomy cannot achieve this.

The present invention seeks to alleviate, at least partially, some or any of the above problems.

The present invention provides a vascular implant as defined in claim 1.

Embodiments of the invention will now be described, by way of example only, with reference to the accompanying drawings in which:
Fig. 1 is a schematic cross-section of an implanted valve to illustrate its operation as an embodiment of a vascular implant according to the invention;
Figs. 2 and 3 are illustrations of a prosthetic heart valve of another embodiment of the invention; and
Fig. 4 shows the heart valve of Figs. 2 and 3 implanted at the location of the aortic valve.

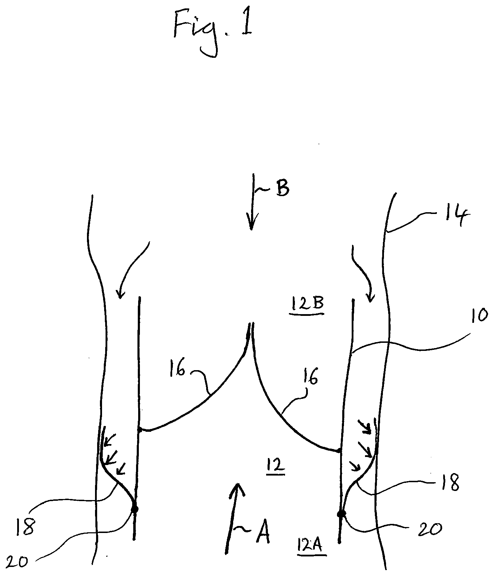

An embodiment of the invention comprising an implantable valve will now be described with reference to the schematic cross-section shown in Fig. 1. In this embodiment, the implant comprises a structure 10 defining a flow passage 12 having a first end 12A and a second end 12B. The structure 10 sits within the native anatomy 14. The structure 10 is generally approximately cylindrical, but in Fig. 1 is, of course, shown in longitudinal cross-section. The structure 10 typically comprises a framework made from a metal tube or wire, but may take any suitable form known in the art.

The structure 10 supports a flow control device, in this case a valve comprising valve leaflets 16 located in the lumen of the flow passage 12. Such valves are well known in the art, and can comprise one, two, three or more leaflets 16.

According to preferred embodiments of the invention, the implant can be, for example, a prosthetic heart valve, such as an aortic valve, pulmonary valve, mitral valve or tricuspid valve.

In normal operation, fluid, in this embodiment blood, can flow from the first end 12A to the second end 12B of the flow passage 12 in the direction indicated by the arrow A because the leaflets 16 are flexible and the leaflets separate to permit flow. Flow in the reverse direction indicated by the arrow B is prevented by the closure by the leaflets 16.

However, with conventional devices, it may be possible for paravalvular leakage to occur by flow of fluid around the exterior of the structure 10 where there are any gaps between the structure and the native anatomy. In Fig. 1, the clearance between the structure 10 and the native anatomy 14 is shown exaggerated for clarity. In practice, the implant will fit much more snugly in the implant site (which also helps anchor the implant in place). However, conventionally, some leakage may be unavoidable due to natural irregularities in the native anatomy and the inability to form a perfect seal all the way round.

As illustrated in Fig. 1, this embodiment of the present invention provides a skirt 18 around the outer periphery of the structure 10. The skirt 18 may also be described as a flap, wing, parachute or similar. The skirt 18 is fixed to the structure 10 at a joining line 20 such that it forms a seal with respect to the flow passage 12.

The joining line 20 can lie in a plane, as in this embodiment, or can take other shapes, regular or irregular. The skirt 18 and the joining line 20 are not limited to being in the particular location relative to the structure 10 illustrated in Fig. 1, but could, for example, be adjacent to the leaflets 16, or could be positioned at either extreme end of the structure 10 or any intermediate position. If the joining line 20 is adjacent to the end of the structure 10 at the second end 12B of the flow passage 12, then the skirt 18 will extend beyond the end of the structure 10.

The skirt 18 is compliant, for example being made of a flexible membrane. Suitable material for the skirt 18 includes biological tissue, polymer, fabric, or a combination thereof. For example, the skirt can be entirely synthetic, formed from artificial polymeric material, or can be biologically-derived, for example a xenograft of bovine pericardium or porcine pericardium, or a combination of synthetic and biologically-derived material. Any other bio-compatible material suitable to be formed into a membrane could be used. The skirt is substantially impermeable to prevent flow of the relevant fluid therethrough, or becomes impermeable after the implant, for example due to thrombus coating or cell proliferation. Although the skirt 18 is flexible, it can be elastic or inelastic. The skirt 18 is fixed to the structure 10 by any suitable technique, such as gluing or suturing or by being made as an integral component of the implant.

Fig. 1 illustrates the situation in which the fluid pressure at the second end of the flow passage 12B is higher than the fluid pressure at the first end of the flow passage 12A. In this case the leaflets 16 of the valve are urged together so that the valve is closed and reverse flow through the flow passage is prevented. If the implant is a replacement aortic valve or pulmonary valve, with the first end 12A at the left or right ventricle, respectively, and the second end 12B at the aorta or pulmonary artery, respectively, then this situation occurs during diastole when the pressure in the outlet artery rises above the ventricular pressure. If the implant is a mitral valve or tricuspid valve, with the first end 12A in the respective atrium and the second end 12B in the respective ventricle, then this situation occurs during systole, when the ventricular pressure exceeds the atrial pressure.

The higher pressure at the end 12B can cause blood to flow around the implant where there are any gaps between it and the native anatomy 14. The fluid pressure then urges the skirt 18 against the native anatomy 14, as indicated by the small arrows in Fig. 1. This engages the skirt 18 against the anatomy at the implant site and occludes any gaps, and results in a seal against leakage. In effect, the skirt 18 acts like a parachute that becomes inflated when there is back pressure against the valve.

The compliance of the material of the skirt 18 means that it can conform to irregularities in the anatomy 14 at the location of the implant. The design of the skirt 18 means that damage to the native anatomy due to excessive radial force is avoided because the force urging the skirt into sealing engagement against the native anatomy 14 is provided by the fluid pressure to which the anatomy would be subjected to anyway in the absence of the implant.

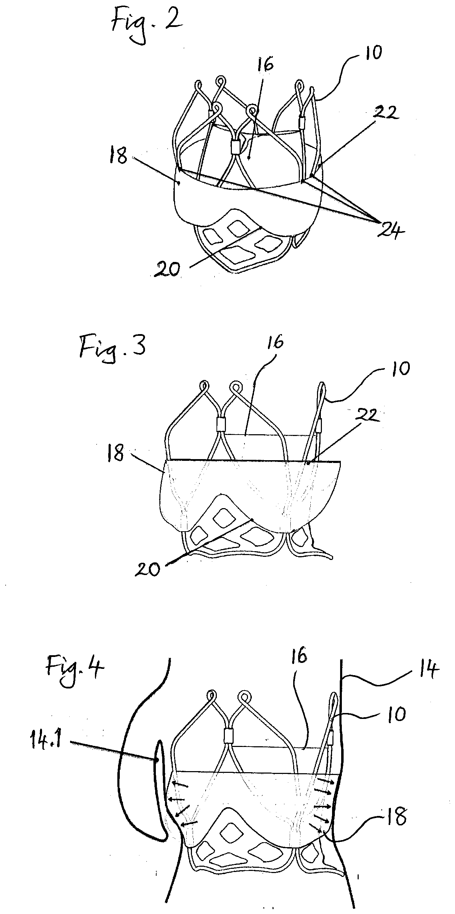

A further specific embodiment of an implant according to the invention is illustrated in Figures 2, 3 and 4. Parts corresponding to those illustrated in Fig. 1 are indicated with corresponding reference numerals, and so repetition of the description of those will be avoided.

The implant of Figures 2 to 4 is a self-expanding prosthetic aortic valve. Further details of its structure can be obtained from WO 2010/112844. The implant has the addition of the skirt 18. As illustrated in Figures 2 and 3, the joining line 20 at the closed edge of the skirt 18 where it is fixed to the structure, is curved such that it is scalloped.

In this embodiment, the open edge 22 of the skirt 18 is fixed at one or more points 24 to the structure 10. This prevents complete reversal of the skirt, either when in use, or when being implanted. Fixing points 24 still enable the skirt 18 to "inflate" like a parachute to seal against the anatomy 14, as illustrated in Fig. 4. Fig. 4 also illustrates that a part of the anatomy around the implant site can be a native valve leaflet 14.1 that is displaced when the prosthetic valve is implanted.

Although a specific implant is illustrated in Figures 2 to 4, which is delivered percutaneously by trans-catheter techniques, the invention is not limited to those particular aspects. The implant in this particular embodiment is self-expanding, but it could equally be expandable or of fixed size.

Although the embodiments described have a single skirt 18 that surrounds the entire implant, an alternative is to have one or more individual skirts. The skirts could, in combination, surround the entire circumference of the implant, either with or without overlap. Alternatively, the or each skirt could be provided only at particular positions that are susceptible to leakage.

## Claims

1. A vascular implant comprising:
a structure (10) defining a flow passage (12), the flow passage (12) having a first end (12A) and a second end (12B); and
a skirt (18) around at least a portion of the outer periphery of the structure (10),
wherein a portion of the skirt (18) is fixed to the structure (10) to form a seal with respect to the flow passage (12), and
**characterised in that** the skirt (18) is compliant such that, in response to a higher fluid pressure at one of the ends of the flow passage (12), the skirt (18) is configured to inflate like a parachute so as to be urged against the anatomy (14) at the site in which the implant is located, to span between the outer periphery of the structure (10) and said anatomy (14).

2. An implant according to claim 1 configured such that fluid can enter between the outer periphery of the structure (10) and the skirt (18) to urge the skirt (18) outward against said anatomy (14).

3. An implant according to claim 1 or 2, wherein the skirt (18) is formed of a membrane.

4. An implant according to any preceding claim, wherein the skirt (18) is made of at least one of: biological tissue, polymer, or fabric.

5. An implant according to any preceding claim, wherein said portion of the skirt (18) fixed to the structure (10) comprises a first edge of the skirt (18) that is sealed with respect to the flow passage (12) around the complete circumference of the structure (10).

6. An implant according to any preceding claim, wherein an edge (22) of the skirt (18), separate from said portion that is fixed to the structure (10), is also attached at one or more locations around the structure (10).

7. An implant according to any preceding claim, wherein the flow passage (12) contains a valve (16) permitting flow in a direction from the first end (12A) to the second end (12B), and restricting flow in the reverse direction, and wherein when the fluid pressure at the second end (12B) is higher than the fluid pressure at the first end (12A), the skirt (18) is urged against the surrounding anatomy (14) at the implant site to restrict paravalvular leakage.

8. An implant according to claim 7 which is a prosthetic heart valve.

9. An implant according to any preceding claim, wherein the skirt (18) is fixed to the structure (10) at a joining line (20) at a position intermediate either end of the structure (10).

10. An implant according to any preceding claim, comprising a plurality of skirts (18) arranged around the outer periphery of the structure (10).

## Patentansprüche

1. Gefässimplantat, umfassend:
eine Struktur (10), die einen Durchfluss (12) definiert, wobei der Durchfluss (12) ein erstes Ende (12A) und ein zweites Ende (12B) aufweist; und
eine Randleiste (18) um mindestens einen Abschnitt des Außenumfangs der Struktur (10),
wobei ein Abschnitt der Randleiste (18) an der Struktur (10) befestigt ist, um eine Versiegelung in Bezug auf den Durchfluss (12) zu bilden, und
**dadurch gekennzeichnet, dass** die Randleiste (18) nachgiebig ist, sodass, in Reaktion auf einen höheren Flüssigkeitsdruck an einem der Enden des Durchflusses (12), die Randleiste (18) konfiguriert ist, sich wie ein Fallschirm aufzublähen, sodass sie an dem Ort, in dem sich das Implantat befindet, gegen die Anatomie (14) gedrängt wird, um sich zwischen dem Außenumfang der Struktur (10) und der Anatomie (14) zu erstrecken.

2. Implantat nach Anspruch 1, so konfiguriert, dass Flüssigkeit zwischen dem Außenumfang der Struktur (10) und der Randleiste (18) eintreten kann, um die Randleiste (18) nach außen gegen die Anatomie (14) zu drängen.

3. Implantat nach Anspruch 1 oder 2, wobei die Randleiste (18) aus einer Membran gebildet ist.

4. Implantat nach einem der vorstehenden Ansprüche, wobei die Randleiste (18) aus mindestens einem der Folgendem hergestellt ist: biologisches Gewebe, Polymer oder einem Gewebe.

5. Implantat nach einem der vorstehenden Ansprüche, wobei der Abschnitt der Randleiste (18), der an der Struktur (10) befestigt ist, eine erste Kante der Randleiste (18) umfasst, die in Bezug zum Durchfluss (12) um den vollständigen Umfang der Struktur (10) versiegelt ist.

6. Implantat nach einem der vorstehenden Ansprüche, wobei eine Kante (22) der Randleiste (18), getrennt vom an der Struktur (10) befestigten Abschnitt, außerdem an einem oder mehreren Orten um die Struktur (10) herum befestigt ist.

7. Implantat nach einem der vorstehenden Ansprüche, wobei der Durchfluss (12) ein Ventil (16) umfasst, dass einen Fluss in eine Richtung vom ersten Ende (12A) zum zweiten Ende (12B) erlaubt, und das einen Fluss in die umgekehrte Richtung einschränkt, und wobei, wenn der Flüssigkeitsdruck am zweiten Ende (12B) höher ist als der Flüssigkeitsdruck am ersten Ende (12A), die Randleiste (18) gegen die umgebende Anatomie (14) am Implantatsort gedrängt wird, um ein paravalvuläres Ausströmen einzuschränken.

8. Implantat nach Anspruch 7, das eine prosthetische Herzklappe ist.

9. Implantat nach einem der vorstehenden Ansprüche, wobei die Randleiste (18) an einer Verbindungslinie (20) an einer Position zwischen jedem Ende der Struktur (10) an der Struktur (10) befestigt ist.

10. Implantat nach einem der vorstehenden Ansprüche, umfassend eine Vielzahl von Randleisten (18), angeordnet um den Außenumfang der Struktur (10).

## Revendications

1. Implant vasculaire comprenant :
une structure (10) délimitant un passage d'écoulement (12), le passage d'écoulement (12) présentant une première extrémité (12A) et une deuxième extrémité (12B), et
une jupe (18) sur au moins une partie de la périphérie extérieure de la structure (10) ;
une partie de ladite jupe (18) étant fixée à la structure (10) afin de former un joint hermétique par rapport au passage d'écoulement (12), et
**caractérisé en ce que** la jupe (18) est flexible si bien que, en réaction à une pression fluidique plus forte au niveau de l'une des extrémités du passage d'écoulement (12), la jupe (18) est conçue de manière à gonfler tel un parachute afin d'être poussée contre la partie anatomique (14) à l'emplacement auquel est situé l'implant, pour s'étendre entre la périphérie extérieure de la structure (10) et ladite partie anatomique (14).

2. Implant selon la revendication 1, conçu de manière que du fluide peut entrer entre la périphérie extérieure de la structure (10) et la jupe (18) pour pousser la jupe (18) vers l'extérieur, contre ladite partie anatomique (14).

3. Implant selon la revendication 1 ou 2, dans lequel la jupe (18) est formée par une membrane.

4. Implant selon l'une quelconque des revendications précédentes, dans lequel la jupe (18) est constituée d'un tissu biologique, d'un polymère et/ou d'un textile.

5. Implant selon l'une quelconque des revendications précédentes, dans lequel ladite partie de la jupe (18) fixée à la structure (10) comprend un premier bord de la jupe (18) formant un joint hermétique par rapport au passage d'écoulement (12) sur toute la circonférence de la structure (10).

6. Implant selon l'une quelconque des revendications précédentes, dans lequel un bord (22) de la jupe (18), séparé de ladite partie fixée à la structure (10), est également rattaché en au moins un endroit situé autour de la structure (10).

7. Implant selon l'une quelconque des revendications précédentes, dans lequel le passage d'écoulement (12) contient une valve (16) permettant un écoulement dans un sens allant de la première extrémité (12A) vers la deuxième extrémité (12B), et limitant l'écoulement dans le sens inverse, et dans lequel, lorsque la pression fluidique régnant au niveau de la deuxième extrémité (12B) est plus élevée que la pression fluidique régnant au niveau de la première extrémité (12A), la jupe (18) est poussée contre la partie anatomique (14) environnante de l'emplacement de l'implant afin de limiter les fuites paravalvulaires.

8. Implant selon la revendication 7, constituant une valve cardiaque prosthétique.

9. Implant selon l'une quelconque des revendications précédentes, dans lequel la jupe (18) est fixée à la structure (10) sur une ligne de jointure (20) en un emplacement intermédiaire aux deux extrémités de la structure (10).

10. Implant selon l'une quelconque des revendications précédentes, comprenant une pluralité de jupes (18) agencées sur la périphérie extérieure de la structure (10).
